(19) Europäisches Patentamt
European Patent Office
Office européen des brevets

(11) Numéro de publication: 0 194 919 B1

# (12) FASCICULE DE BREVET EUROPEEN

(45) Date de publication du fascicule du brevet: **18.07.90**

(51) Int. Cl.⁵: **C07C 69/743**, C07C 255/32, C07D 213/64, C07D 233/72, A01N 53/00, A23K 1/16

(21) Numéro de dépôt: **86400428.8**

(22) Date de dépôt: **28.02.86**

(54) **Nouveaux esters dérivés de l'acide 2,2-diméthyl 3-éthényl cyclopropane carboxylique, leur préparation, leur application à la lutte contre les parasites et les compositions les renfermant.**

(30) Priorité: **01.03.85 FR 8503037**

(43) Date de publication de la demande: **17.09.86 Bulletin 86/38**

(45) Mention de la délivrance du brevet: **18.07.90 Bulletin 90/29**

(84) Etats contractants désignés: **BE CH DE GB IT LI LU NL**

(56) Documents cités:
**EP-A- 0 037 851**
**EP-A- 0 061 053**
**DE-A- 2 826 864**
**FR-A- 2 084 816**
**FR-A- 2 143 820**

**Le dossier contient des informations techniques présentées postérieurement au dépôt de la demande et ne figurant pas dans le présent fascicule.**

(73) Titulaire: **ROUSSEL-UCLAF, 35, boulevard des Invalides, F-75007 Paris(FR)**

(72) Inventeur: **Tessier, Jean, 30, Rue Jean Moulin, F-94300 Viencennes(FR)**
Inventeur: **Demoute, Jean-Pierre, 249bis, Rue de Rosny, F-93100 Montreuil-sous-Bois(FR)**

(74) Mandataire: **Tonnellier, Marie-José et al, Département des Brevets ROUSSEL UCLAF B.P. no 9, F-93230 Romainville(FR)**

EP 0 194 919 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux esters dérivés de l'acide 2,2-diméthyl 3-éthényl cyclopropane carboxylique, leur préparation, leur application à la lutte contre les parasites et les compositions les renfermant.

L'invention a pour objet les composés répondant à la formule générale (I) :

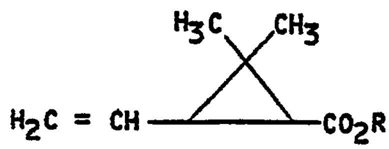

(I)

ans laquelle R représente :

a/. un radical :

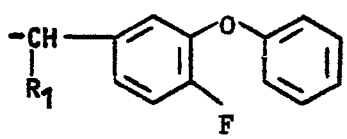

dans lequel $R_1$ représente un atome d'hydrogène, un radical $-CH_3$, $-C{\equiv}N$ ou $-C{\equiv}CH$,

b/. un radical :

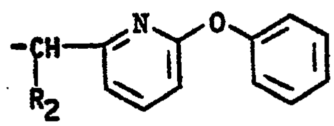

dans lequel $R_2$ représente un atome d'hydrogène, un radical $-CH_3$, $-C{\equiv}N$ ou $-C{\equiv}CH$,

c/. un radical :

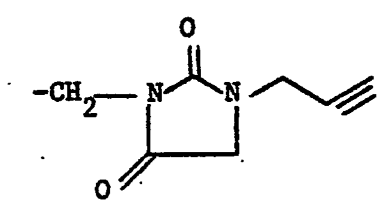

sous toutes les formes isomères possibles ainsi que tous les mélanges d'isomères possibles.

Les composés de formule I peuvent exister sous diverses formes stéréoisomères : ils possèdent, en effet, deux carbones asymétriques en position 1 et 3 du cyclopropane : ils peuvent posséder également un ou plusieurs centres d'asymétrie dans la partie R.

L'invention a plus particulièrement pour objet les composés de formule I dans lesquels la copule cyclopropanique est de structure (1R)cis ou (1R)trans.

Parmi les composés de l'invention, on peut citer notamment les composés décrits dans la partie expérimentale.

Comme composés préférés de l'invention, on peut citer tout particulièrement les composés répondant à la formule $I_A$ :

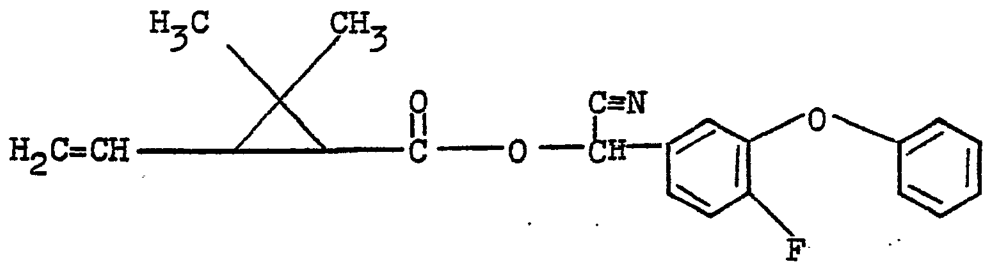

( $I_A$ )

et notamment le 1R,cis 2,2-diméthyl 3-éthényl cyclopropane carboxylate de (S) cyano (3-phénoxy 4-fluoro phényl) méthyle.

Les composés de formule I font partie de la famille des esters de l'acide 2,2-diméthyl 3-éthényl cyclopropane carboxylique, qui sont connus d'une façon générale : certains composés de cette famille sont décrits notamment dans le brevet français 2 143 820 et dans les documents EP-A 0 037 851, EP-A 0 061 053, DE-A 2 826 864 et FR-A 2 084 816.

On vient de découvrir que certains composés de cette famille, à savoir les composés répondant à la formule I, qui n'ont jamais été décrits à ce jour, possédaient des propriétés pesticides exceptionnelles, très supérieures à celles des produits chimiques les plus proches comme le montrent les résultats des tests biologiques décrits ci-après dans la partie expérimentale.

L'invention a également pour objet un procédé de préparation des composés de formule I caractérisé en ce que l'on soumet un acide de formule (II) :

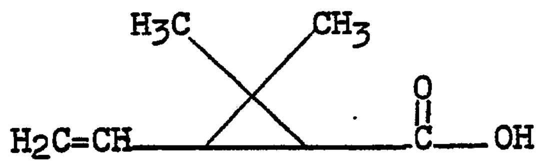

(II)

ou un dérivé fonctionnel de cet acide à l'action d'un alcool de formule III :

R-OH (III)

dans laquelle R conserve la même signification que précédemment pour obtenir le composé de formule I correspondant.

Par dérivé fonctionnel d'acide, on entend de préférence un chlorure ou un anhydride d'acide.

Selon un mode d'exécution préféré du procédé de l'invention, on estérifie l'acide II par l'alcool III en présence de dicyclohexyl-carbodiimide et de 4-diméthyl aminopyridine.

Les produits de formule I peuvent également être préparés selon les autres procédés d'estérification décrits dans la littérature.

Les acides de formule II sont des produits connus, décrits, par exemple, par ORTIZ de MONTELLANNO dans J. ORG. CHEM. 1978, 43, p 4 323-8.

Les alcools de formule III sont des produits connus, décrits, par exemple, dans les brevets français 2 382 426, 2 415 105, 2 383 927.

Les composés de formule (I) présentent d'intéressantes propriétés qui permettent leur utilisation dans la lutte contre les parasites. Il peut s'agir, par exemple, de la lutte contre les parasites des végétaux, les parasites des locaux et les parasites des animaux à sang chaud.

C'est ainsi que l'on peut utiliser les composés de l'invention pour lutter contre les insectes, les nématodes et les acariens parasites des végétaux, des locaux et des animaux.

L'invention a donc ainsi pour objet l'application des composés de formule I à la lutte contre les parasites des végétaux, les parasites des locaux et les parasites des animaux à sang chaud.

Les produits de formule (I) peuvent être utilisés notamment pour lutter contre les insectes dans le domaine agricole, pour lutter, par exemple, contre les pucerons, les larves de lépidoptères et les coléoptères. Ils sont utilisés à des doses comprises entre 10 g et 300 g de matière active à l'hectare.

Les produits de formule (I) peuvent également être utilisés pour lutter contre les insectes dans les locaux, pour lutter notamment contre les mouches, les moustiques et les blattes, ainsi que pour lutter contre les insectes parasites des animaux, par exemple les poux, notamment chez les bovins, les ovins et les volailles.

Les produits de formule (I) présentent un pouvoir d'abattage exceptionnel comme le montrent les résultats des tests joints en annexe.

Les produits de formule (I) peuvent aussi être utilisés pour lutter contre les acariens et les nématodes parasites des végétaux.

Les composés de formule (I) peuvent encore être utilisés pour lutter contre les acariens parasites des animaux, pour lutter par exemple contre les tiques et notamment les tiques de l'espèce Boophilus, ceux de l'espèce Hyalomnia, ceux de l'espèce Amblyomnia et ceux de l'espèce Rhipicephalus ou pour lutter contre toutes sortes de gales et notamment la gale sarcoptique, la gale psoroptique et la gale chorioptique.

L'invention a aussi pour objet les compositions destinées à la lutte contre les parasites des végétaux, les parasites des locaux et les parasites des animaux à sang chaud, caractérisées en ce qu'elles renferment comme principe actif au moins un des produits définis précédemment.

L'invention a notamment pour objet les compositions insecticides renfermant comme principe actif au moins l'un des produits définis précédemment.

L'invention a également pour objet les compositions acaricides renfermant comme principe actif au moins l'un des produits définis précédemment ainsi que les compositions nématicides renfermant comme principe actif au moins l'un des produits définis précédemment.

L'invention a également pour objet les compositions acaricides destinées à la lutte contre les acariens parasites des animaux à sang chaud, notamment contre les tiques et les gales, caractérisées en ce qu'elles renferment comme principe actif l'un au moins des produits définis ci-dessus.

Parmi les compositions pesticides préférées de l'invention, on peut citer tout spécialement les compositions renfermant les composés décrits dans les exemples.

Les compositions selon l'invention sont préparées selon les procédés usuels de l'industrie agrochimique. Elles peuvent être additionnées éventuellement d'un ou plusieurs autres agents pesticides.

Ces composi tions peuvent se présenter sous forme de poudres, granulés, suspensions, émulsions, solutions, solutions pour aérosols, bandes combustibles, appâts ou autres préparations employés classiquement pour l'utilisation de ce genre de composés.

Outre le principe actif, ces compositions contiennent, en général, un véhicule et/ou un agent tensioactif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005 à 10 % en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin combustible, ou encore d'un substrat fibreux incombustible. Dans ce dernier cas, le fumigant obtenu après incorporation de la matière active est placé sur un appareil chauffant.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin), amidon et poudre de coque de noix de coco.

La dose de matière active peut alors être, par exemple, de 0,03 à 1 % en poids.

Dans le cas où l'on utilise un support fibreux incombustible, la dose de matière active peut alors être, par exemple de 0,03 à 25 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 25 % en poids.

Les compositions acaricides et nématicides peuvent se présenter notamment sous forme de poudres, granulés, suspensions, émulsions, solutions.

Pour l'usage acaricide, on utilise de préférence des poudres mouillables, pour pulvérisation foliaire, contenant de 1 à 80% de principe actif ou des liquides pour pulvérisation foliaire contenant de 1 à 500 g/l de principe actif. On peut également employer des poudres pour poudrage foliaire contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Lorsqu'il s'agit de lutter contre les acariens parasites des animaux, on peut incorporer les produits de l'invention dans des compositions alimentaires en association avec un mélange nutritif adapté à l'alimentation animale. Le mélange nutritiel peut varier selon l'espèce animale, il peut renfermer des céréales, des sucres et des grains, des tourteaux de soja, d'arachide et de tournesol, des farines d'origine animale, par exemple des farines de poissons, des acides aminés de synthèse, des sels minéraux, des vitamines et des antioxydants.

L'invention a donc ainsi également pour objet les compositions destinées à l'alimentation animale renfermant comme principe actif au moins l'un des produits définis précédemment.

Lorsqu'il s'agit de lutter contre les acariens parasites des animaux, les compositions de l'invention peuvent être administrées par voie externe, par vaporisation, par shampooing, par bain ou badigeonnage de l'épine dorsale selon la méthode "pour-on".

L'invention a également pour objet les associations douées d'activité insecticide, acaricide ou nématicide, caractérisées en ce qu'elles contiennent comme matière active d'une part un au moins des composés de formule générale (I) et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxybenzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcool α-cyano 3-phénoxy benzyliques des acides 2,2-diméthyl 3-(2,2-dichlorovinyl) cylopropane-1-carboxyliques, par les esters d'alcools α-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones, d'alcool 3,4,5,6-tétrahydro-phtalimido méthylique, d'alcool 5-benzyl 3-furyl méthylique, d'alcool 3-phénoxy benzylique et d'alcool α-cyano 3-phénoxy benzyliques des acides 2,2-diméthyl 3-(1,2,2,2-tétrahaloéthyl) cyclopropane-1-carboxyliques, dans lesquels "halo" représente un atome de fluor, de chlore ou de brome, étant entendu que les copules acides et alcools des esters pyréthrinoïdes ci-dessus peuvent exister sous toutes leurs formes stéréoisomères possibles.

Pour exalter l'activité biologique des produits de l'invention, on peut les additionner à des synergistes classiques utilisés en pareil cas tels que le 1-(2,5,8-trioxadodécyl) 2-propyl 4,5-méthylène dioxy benzène (ou butoxyde de pipéronyle) ou la N-(2-éthylheptyl) bicyclo (2,2-1)-5-heptène 2,3-d1carboximide ou le pipéronyl-bis-2-(2'-n butoxyéthoxy) éthylacétal (ou tropital).

L'invention a donc également pour objet les compositions et associations telles que définies précédemment, caractérisées en ce qu'elles renferment, en outre, un synergiste des pyréthrinoïdes.

Les exemples suivants illustrent l'invention sans toutefois la limiter.

Exemple 1 : 1R, cis 2,2-diméthyl 3-éthényl cyclopropane carboxylate de S-cyano (3-phénoxy 4-fluoro phényl) méthyle.

On mélange 3,5 g d'acide 1 R,cis 2,2-diméthyl 3-éthényl cyclopropane carboxylique, 35 cm3 de chlorure de méthylène, 50 mg de 4-diméthyl amino pyridine, 6,44 g de S-cyano (3-phénoxy 4-fluoro phényl) méthanol, introduit à 0° C une solution de 5,16 g de dicyclohexyl carbodiimide dans 10 cm3 de chlorure de méthylène, agite pendant 1 heure à 0°C, puis pendant 6 heures à 20°C, filtre, concentre le filtrat à sec par distillation sous pression réduite, chromatographie le résidu sur silice en éluant par un mélange d'hexane et d'acétate d'éthyle (8/2) et obtient 6,35 g de produit attendu.

Rf = 0,45 $/\alpha/_D$ = +32,5° (c = 1%, chloroforme).

Exemple 2 : 1R, trans 2,2-diméthyl 3-éthényl cyclopropane carboxylate de (S) cyano (4-fluoro 3-phénoxy phényl) méthyle.

On mélange 1 g d'acide 1R, trans 2,2-diméthyl 3-éthényl cyclopropane carboxylique, 10 cm3 de chlorure de méthylène, 1,75 g de (S) cyano (4-fluoro 3-phénoxy phényl) méthanol, 0,05 g de 4-diméthyl amino pyridine, introduit à 0°C, une solution de 1,48 g de dicyclohexyl-carbodiimide dans 5 cm3 de chlorure de méthylène, agite pendant 4 heures à 20°C, filtre, concentre le filtrat à sec par distillation sous pression réduite, chromatographie le résidu sur silice en éluant avec un mélange d'hexane et d'acétate d'éthyle (8/2) et obtient 1,35 g de produit attendu Rf = 0,4, $/\alpha/_D$ = -2,5° (c = 1,5 % chloroforme).

Exemples 3 à 6 : En opérant comme à l'exemple 1 et 2, à partir des acides et des alcools correspondants, on a préparé les produits suivants :

Exemple 3 : 1R,cis 3-éthényl 2,2-diméthyl cyclopropane carboxylate d'α (RS) cyano (6-phénoxy pyrid-2-yl) méthyle.

$/\alpha/_D$ = +22° ± 1° c = 0,2% $CHCl_3$.

Exemple 4 : 1R,cis 3-éthényl 2,2-diméthyl cyclopropane carboxylate de /(3 propyn-2-yl) 2,5-dioxo imidazolidinyl/ méthyle.

$/\alpha/_D$ = +13° ± 1° c = 0,5% $CHCl_3$.

Exemple 5 : 1R,trans 3-éthényl 2,2-diméthyl cyclopropane carboxylate d'α (RS) cyano (6-phénoxy pyrid-2-yl) méthyle.

$/\alpha/_D$ = +7° ± 2° c = 0,5% pyridine.

Exemple 6 : 1R,trans, 3-éthényl 2,2-diméthyl cyclopropane carboxylate de /(3-propyn-2 yl) 2,5-dioxo imidazolidinyl/ méthyle.

$/\alpha/_D$ = -3° ± 1,5° c = 1 % pyridine.

Exemple 7 : Préparation d'un concentré soluble :

On effectue un mélange homogène de :
Produit de l'exemple 1 .............................. 0,25 g
Butoxyde de pipéronyle .............................. 1,00 g
Tween 80 ............................................ 0,25 g
Topanol A ........................................... 0,1 g
Eau ................................................. 98,4 g

Exemple 8 : Préparation d'un concentré émulsifiable :

On mélange intimement :
Produit de l'exemple 3 .............................. 0,005 g
Butoxyde de pipéronyle .............................. 0,05 g
Topanol A ........................................... 0,1 g
Tween 80 ................... ........................ 3,5 g
Xylène .............................................. 96,345 g

Exemple 9 : Préparation d'un concentré émulsifiable :

On effectue un mélange homogène de :
Produit de l'exemple 4 .............................. 10 g
Tween 80 ............................................ 15 g
Topanol A ........................................... 0,5 g
Xylène .............................................. 74,5 g

Exemple 10 : Préparation d'une composition fumigène :

On mélange d'une façon homogène :
Produit de l'exemple 3 .............................. 0,25 g
Poudre de Tabu ...................................... 25,00 g
Poudre de feuille de cèdre .......................... 40,00 g
Poudre de bois de pin ............................... 33,75 g
Vert brillant ....................................... 0,5 g
p-nitrophénol ....................................... 0,5 g

Exemple 11 : Etude biologique des composés de l'invention :

a/ Etude de l'effet d'abattage sur mouche domestique.

Les insectes tests sont des mouches domestiques femelles âgées de 4 à 5 jours. On opère par pulvérisation directe à la concentration de 0,100 g/l en cylindre de Kearns et March en utilisant comme solvant un mélange d'acétone (5 %) et d'Isopar L (solvant pétrolier) (quantité de solvant utilisée:2 ml en une seconde). On utilise 50 insectes par traitement. On effectue les contrôles d'abattage toutes les minutes jusqu'à 10 minutes, puis à 15 minutes et l'on détermine le KT 50 par les méthodes habituelles.

Les produits ont été étudiés comparativement à deux produits analogues dérivés de l'alcool (S) cyano /3-phénoxy phényl/ méthyle, à savoir le 1R,cis 2,2-diméthyl 3-éthényl cyclopropane carboxylate de cyano /3-phénoxy phényl) méthyle (Produit $P_1$), et le 1R,trans 2,2-diméthyl 3-éthényl cyclopropane carboxylate de cyano /3-phénoxy phényl/ méthyle (Produit $P_2$). Le produit de référence est la bioalléthrine utilisée à 0,100 g/l.

Les résultats obtenus exprimés en puissance relative par rapport a la bioalléthrine sont les suivants :

| Produits | KT 50 exprimé en puissance relative par rapport à la bioalléthrine |
|---|---|
| Produit de l'exemple 1 | 7,3 |
| Produit de l'exemple 3 | 9,2 |
| Produit de l'exemple 4 | 9,8 |
| Produit de l'exemple 5 | 7,4 |
| Produit de l'exemple 6 | 6,8 |
| Produit $P_1$ | 2,8 |
| Produit $P_2$ | 1,8 |

b/. Etude de l'effet létal sur chenilles de Spodoptera Littoralis.

Les essais sont effectués par application topique d'une solution acétonique toxique à l'aide du micro manipulateur d'Arnold sur le thorax dorsal des chenilles; On détermine la $DL_{50}$ du produit en utilisant 15 larves par dose de produit à tester. Les chenilles utilisées sont des larves du quatrième stade larvaire, c'est-à-dire âgées d'environ 10 jours en élevage sur milieu nutritif artificiel (milieu de Poitou) à 24°C et 65% d'humidité relative. Après traitement, les individus sont mis en observation sur milieu nutritif.

On effectue le contrôle des mortalités 48 heures après traitement.

Le produit de l'exemple 1 a été étudié comparativement au produit analogue dérivé de l'alcool S-cyano /3-phénoxy phényl/ méthyle (produit $P_1$). On a trouvé que le produit de l'exemple 1 était deux à trois fois plus actif que le produit $P_1$.

## Revendications

1 - Les composés répondant à la formule générale I :

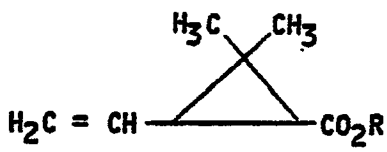

(I)

dans laquelle R représente :

a/. un radical :

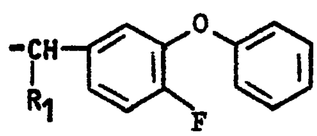

dans lequel $R_1$ représente un atome d'hydrogène, un radical $-CH_3$, $-C{\equiv}N$ ou $-C{\equiv}CH$,

b/. un radical :

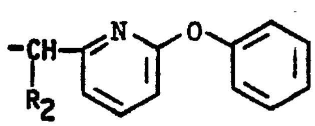

dans lequel $R_2$ représente un atome d'hydrogène, un radical $-CH_3$, $-C{\equiv}N$ ou $-C{\equiv}CH$, ou

c/. un radical :

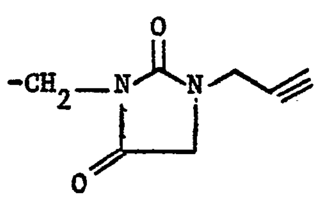

sous toutes les formes isomères possibles, ainsi que tous les mélanges d'isomères possibles.

2 - Les composés de formule I telle que définie à la revendication 1, dans lesquels la copule cyclopropanique est de structure 1R,cis ou 1R,trans.

3 - Les composés tels que définis à la revendication 1 ou 2 répondant à la formule ($I_A$) :

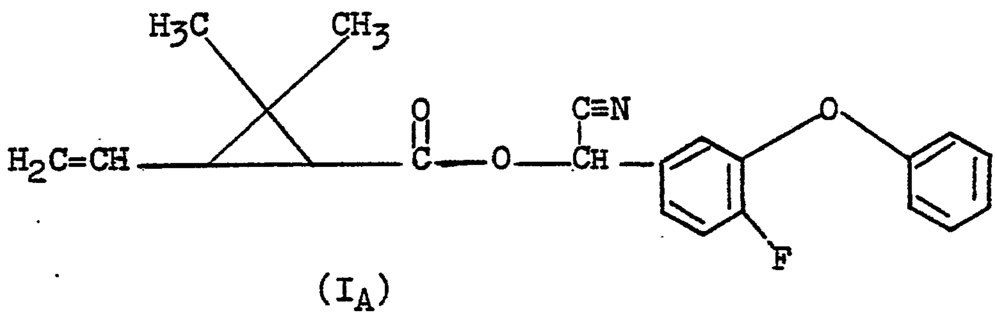

($I_A$)

4 - Le 1R,cis 2,2-diméthyl 3-éthényl cyclopropane carboxylate de (S) cyano (3-phénoxy 4-fluoro phényl) méthyle.

5 - Procédé de préparation des composés de formule I tels que définis à la revendication 1, caractérisé en ce que l'on soumet un acide de formule II :

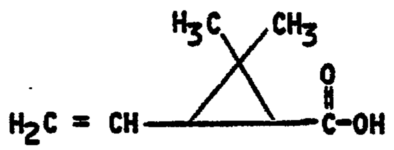

(II)

ou un dérivé fonctionnel de cet acide à l'action d'un alcool de formule III :

R-OH (III)

dans laquelle R conserve la même signification que précédemment, pour obtenir le composé de formule I correspondant.

6 - Application des composés de formule I tels que définis à l'une des revendications 1 à 4, à la lutte contre les parasites des végétaux, les parasites des locaux et les parasites des animaux à sang chaud.

7 - Les compositions destinées à la lutte contre les parasites des végétaux, les parasites des locaux et les parasites des animaux à sang chaud, caractérisées en ce qu'elles renferment comme principe actif au moins un des produits définis à l'une quelconque des revendications 1 à 4.

8 - Les compositions insecticides renfermant comme principe actif au moins l'un des produits définis à l'une quelconque des revendications 1 à 4.

9 - Les compositions acaricides renfermant comme principe actif au moins l'un des produits définis à l'une quelconque des revendications 1 à 4.

10 - Les compositions nématicides renfermant comme principe actif au moins l'un des produits définis à l'une quelconque des revendications 1à 4.

11 - Les compositions acaricides destinées à la lutte contre les parasites des animaux à sang chaud, notamment contre les tiques et les gales, caractérisées en ce qu'elles renferment comme principe actif, au moins l'un des produits définis à l'une quelconque des revendications 1 à 4.

12 - Les compositions destinées à l'alimentation animale renfermant comme principe actif au moins l'un des produits définis à l'une quelconque des revendications 1 à 4.

13 - Associations douées d'activité insecticide, acaricide ou nématicide, caractérisées en ce qu'elles contiennent comme matière active, d'une part un au moins des composés de formule générale (I) et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools α-cyano 3-phénoxy benzyliques des acides 2,2-diméthyl 3-(2,2-dichlorovinyl) cyclopropane-1-carboxyliques, par les esters d'alcools α -cyano 3-phénoxy benzyliques d'acide 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides 2,2-diméthyl 3-(1,2-2,2-tétrahaloéthyl) cyclopropane-1-carboxyliques, dans lesquels "halo" représente un atome de fluor, de chlore ou de brome, étant entendu que les copules acides et alcools des esters pyréthrinoïdes ci-dessus peuvent exister sous toutes leurs formes stéréoisomères possibles.

14 - Les compositions et associations selon l'une quelconque des revendications 7 à 13 caractérisées en ce qu'elles renferment, en outre, un synergiste des pyréthrinoïdes.

**Claims**

1. The compounds corresponding to general formula I:

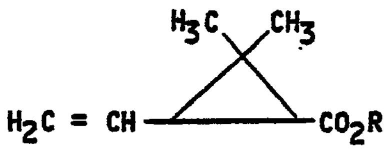

(I)

in which R represents:

a) a radical:

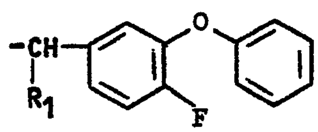

in which $R_1$ represents a hydrogen atom, a $-CH_3$, $-C{\equiv}N$ or $-C{\equiv}CH$ radical,

b) a radical

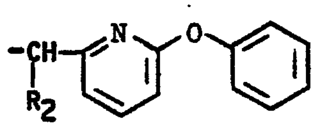

in which $R_2$ represents a hydrogen atom, a $-CH_3$, $-C{\equiv}N$ or $-C{\equiv}CH$ radical,

or

c) a radical

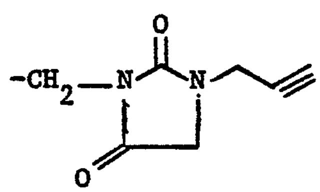

in all the possible isomer forms, as well as all the possible mixtures of isomers.

2. The compounds of formula I as defined in claim 1, in which the cyclopropane copula is of a 1R,cis or 1R,trans structure.

3. The compounds as defined in claim 1 or 2 corresponding to the general formula ($I_A$):

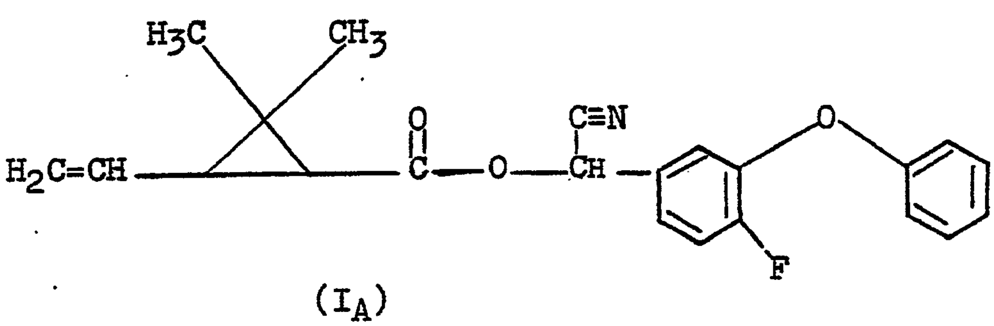

($I_A$)

4. The 1R,cis-2,2-dimethyl-3-ethenyl-cyclopropane carboxylate of (S)-cyano-(3-phenoxy-4-fluorophenyl)-methyl.

5. The preparation process of compounds of formula I as defined in claim 1, characterised in that an acid of formula II:

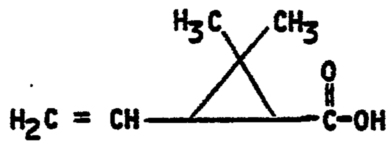

(II)

or a functional derivative of this acid is subjected to the action of an alcohol of formula III:

R–OH (III)

in which R retains the same meaning as previously, so as to obtain the corresponding compound of formula I.

6. The application of compounds of formula I as defined in one of claims 1 to 4, for combating parasites of vegetation, parasites of premises and parasites of warm-blooded animals.

7. The compositions intended to combat parasites of vegetation, parasites of premises and parasites of warm-blooded animals, characterised in that they contain as active principle at least one of the products defined in any one of claims 1 to 4.

8. The insecticide compositions containing as active principle at least one of the products defined in any one of claims 1 to 4.

9. The acaricide compositions containing as active principle at least one of the products defined in any one of claims 1 to 4.

10. The nematicide compositions containing as active principle at least one of the compounds defined in any one of claims 1 to 4.

11. The acaricide compositions intended to combat parasites of warm-blooded animals, in particular ticks and mites, characterised in that they contain as active principle at least one of the products defined in any one of claims 1 to 4.

12. The compositions intended for animal fodder containing as active principle at least one of the products defined in any one of claims 1 to 4.

13. Combinations endowed with an insecticide, acaricide or nematicide activity, characterised in that they contain as active material, on the one hand at least one of the compounds of general formula (I) and on the other hand, at least one of the pyrethrinoid esters chosen from the group constituted by the esters of allethrone, of 3,4,5,6-tetrahydrophtalimidomethyl alcohol, of 5-benzyl-3-furyl methyl alcohol, of 3-phenoxybenzyl alcohol and of alpha-cyano-3-phenoxybenzyl alcohols with chrysanthemic acids, by the esters of 5-benzyl-3-furyl methyl alcohols with 2,2-dimethyl-3-(2-oxo-3-tetrahydrothiophenylidene-methyl)-cyclopropane-1-carboxylic acids, by the esters of 3-phenoxybenzyl alcohol and of alpha-cyano-3-phenoxybenzyl alcohols with 2,2-dimethyl-3-(2,2-dichlorovinyl)-cyclopropane-1-carboxylic acids, by the esters of alpha-cyano-3-phenoxy-benzyl alcohol with 2,2-dimethyl-3-(2,2-dibromovinyl)-cyclopropane-1-carboxylic acids, by the esters of 3-phenoxybenzyl alcohols with 2-parachlorophenyl-2-isopropyl acetic acids, by the allethrolone esters, of 3,4,5,6-tetrahydrophtalimidomethyl alcohol, of 5-benzyl-3-furyl methyl alcohol, of 3-phenoxybenzyl alcohol, and of alpha-cyano-3-phenoxybenzyl alcohols with 2,2-dimethyl-3-(1,2,2,2-tetrahaloethyl)-cyclopropane-1-carboxylic acid, in which "halo" represents a fluorine, chlorine or bromine atom, it being understood that the acid and alcohol copulas of the above pyrethrinoid esters can exist in all their possible stereoisomer forms.

14. The compositions and associations according to any one of claims 7 to 13 characterised in that they contain, moreover, a pyrethrinoid synergist.

**Patentansprüche**

1. Verbindungen der allgemeinen Formel I

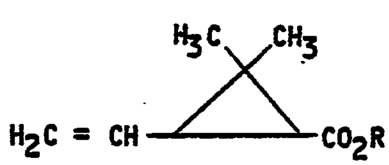

(I)

worin R bedeutet:

(a) einen Rest

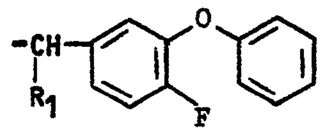

worin $R_1$ für ein Wasserstoffatom, einen Rest $-CH_3$, $-C\equiv N$ oder $-C\equiv CH$ steht;
(b) einen Rest

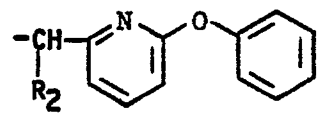

worin $R_2$ für ein Wasserstoffatom, einen Rest $-CH_3$, $-C\equiv N$ oder $-C\equiv CH$ steht;
oder
(c) einen Rest

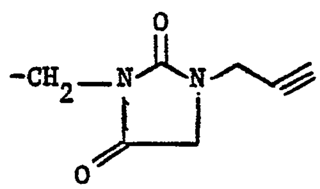

in sämtlichen ihrer möglichen isomeren Formen sowie die Gemische der möglichen Isomeren.

2. Verbindungen der Formel I gemäß Anspruch 1, worin die Cyclopropan-Verknüpfung die 1R,cis- oder 1R,trans-Struktur besitzt.

3. Verbindungen gemäß Anspruch 1 oder 2 der Formel ($I_A$)

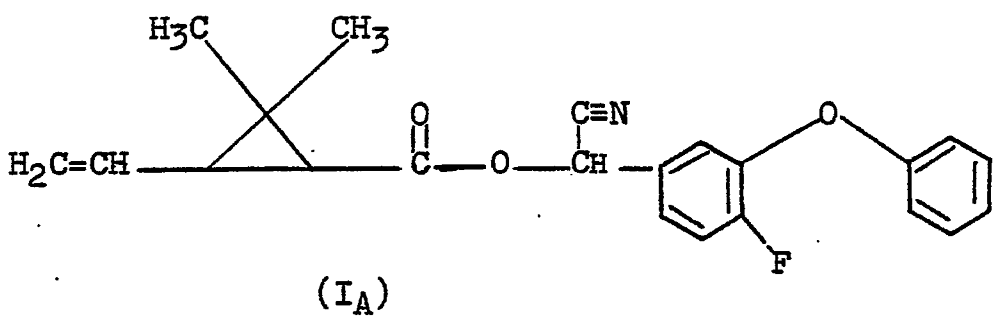

($I_A$)

4. (S)-Cyano-(3-phenoxy-4-fluorphenyl)-methyl-1R,cis-2,2-dimethyl-3-ethenyl-cyclopropancarboxylat.

5. Verfahren zur Herstellung der Verbindungen der Formel I gemäß Anspruch 1, dadurch gekennzeichnet, daß man eine Säure der Formel II

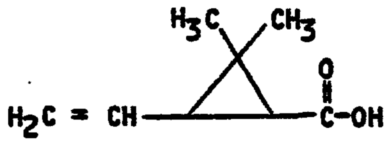

(II)

oder ein funktionelles Derivat dieser Säure der Einwirkung eines Alkohols der Formel III

R–OH (III)

unterzieht, worin R die vorstehend angegebene Bedeutung besitzt, um zu einer entsprechenden Verbindung der Formel I zu gelangen.

6. Verwendung der Verbindungen der Formel I gemäß einem der Ansprüche 1 bis 4 zur Bekämpfung von Parasiten der Pflanzen, Parasiten von Räumlichkeiten und Parasiten warmblütiger Tiere.

7. Zusammensetzungen für die Bekämpfung von Parasiten der Pflanzen, Parasiten von Räumlichkeiten und Parasiten warmblütiger Tiere, dadurch gekennzeichnet, daß sie als Wirkstoff zumindest eines der Produkte gemäß einem der Ansprüche 1 bis 4 enthalten.

8. Insektizide Zusammensetzungen, enthaltend als Wirkstoff zumindest eines der Produkte gemäß einem der Ansprüche 1 bis 4.

9. Akarizide Zusammensetzungen, enthaltend als Wirkstoff zumindest eines der Produkte gemäß einem der Ansprüche 1 bis 4.

10. Nematizide Zusammensetzungen, enthaltend als Wirkstoff zumindest eines der Produkte gemäß einem der Ansprüche 1 bis 4.

11. Akarizide Zusammensetzungen für die Bekämpfung von Parasiten warmblütiger Tiere, insbesondere von Zecken und Krätzmilben, dadurch gekennzeichnet, daß sie als Wirkstoff zumindest eines der Produkte gemäß einem der Ansprüche 1 bis 4 enthalten.

12. Zusammensetzungen für die tierische Ernährung, enthaltend als Wirkstoff zumindest eines der Produkte gemäß einem der Ansprüche 1 bis 4.

13. Mit insektizider, akarizider oder nematizider Aktivität versehene Assoziationen, dadurch gekennzeichnet, daß sie als Wirkstoff einesteils zumindest eine der Verbindungen der allgemeinen Formel (I) und anderenteils zumindest einen Pyrethrinoidester, ausgewählt unter den Estern der Allethrolone, des 3,4,5,6-Tetrahydrophthalimido-methylalkohols, des 5-Benzyl-3-furyl-methylalkohols, des 3-Phenoxybenzylalkohols und der α-Cyano-3-phenoxybenzylalkohole der Chrysanthemumsäuren, unter den Estern des 5-Benzyl-3-furyl-methylalkohols der 2,2-Dimethyl-3-(2-oxo-3-tetrahydrothiophenylidenmethyl)-cyclopropan-1-carbonsäuren, unter den Estern des 3-Phenoxy-benzylalkohols und der α-Cyano-3-phenoxy-benzylalkohole der 2,2-Dimethyl-3-(2,2-dichlorvinyl)-cyclopropan-1-carbonsäuren, unter den Estern der α-Cyano-3-phenoxy-benzylalkohole der 2,2-Dimethyl-3-(2,2-dibromvinyl)-cyclopropan-1-carbonsäuren, unter den Estern des 3-Phenoxy-benzylalkohols der 2-p-Chlorphenyl-2-isopropylessigsäuren, unter den Estern der Allethrolone, des 3,4,5,6-Tetrahydrophthalimidomethylalkohols, des 5-Benzyl-3-furyl-methylalkohols, des 3-Phenoxy-benzylalkohols und der α-Cyano-3-phenoxy-benzylalkohole der 2,2-Dimethyl-3-(1,2,2,2-tetrahaloethyl)-cyclopropan-1-carbonsäuren, enthalten, worin "halo" für ein Fluor-, Chlor- oder Bromatom steht, wobei die sauren Verknüpfungskomponenten und die Alkohole der vorstehend Pyrethrinoidester in sämtlichen ihrer möglichen stereoisomeren Formen vorliegen können.

14. Zusammensetzungen und Assoziationen gemäß einem der Ansprüche 7 bis 13, dadurch gekennzeichnet, daß sie außerdem einen Synergisten der Pyrethrinoide enthalten.